# EUROPEAN PATENT APPLICATION

(11) **EP 4 571 300 A1**
(43) Date of publication of application: **18.06.2025**
(21) Application number: 23852424.3
(22) Date of filing: 01.08.2023
(51) Int. Cl.: G01N 27/62, G01N 33/483

(54) **MATRIX SOLUTION, MASS SPECTROMETRY METHOD, STORAGE MEDIUM, AND DETERMINATION METHOD**

(30) Priority: 12.08.2022 JP 2022128831
(71) Applicant: SHIMADZU CORPORATION, Kyoto-shi, Kyoto 604-8511 (JP)
(72) Inventor: TERAMOTO, Kanae, Kyoto-shi, Kyoto 604-8511 (JP)
(74) Representative: Müller-Boré & Partner Patentanwälte PartG mbB
(86) International application number: PCT/JP2023/028052
(87) International publication number: WO 2024/034453

(57) **Abstract**

A matrix solution used to be mixed with a sample in a matrix-assisted laser desorption/ionization mass spectrometry method. The matrix solution is an aqueous solution containing acetonitrile, ethanol, trifluoroacetic acid, and water. A content of the acetonitrile is 30 to 40 vol% based on the matrix solution. A content of the ethanol is 10 to 20 vol% based on the matrix solution. A content of the trifluoroacetic acid is 1 to 3 vol% based on the matrix solution. The matrix solution contains α-cyano-4-hydroxycinnamic acid, 2,5-dihydroxybenzoic acid, or sinapinic acid in a ratio of 5 to 20 mg/ml.

## Description

### TECHNICAL FIELD

The present invention relates to a matrix solution, a mass spectrometry method, a storage medium, and a determination method, and more particularly, relates to a matrix solution, a mass spectrometry method, a storage medium, and a determination method related to microorganism determination employing a matrix-assisted laser desorption/ionization mass spectrometry method.

### BACKGROUND ART

There is a known method for determining a microorganism based on a pattern of a mass spectrum obtained by mass spectrometry of the microorganism. In particular, among methods of the mass spectrometry, a method for determining a microorganism by employing a matrix-assisted laser desorption/ionization mass spectrometry method (MALDI-MS), which is disclosed in NPLs 1 to 3, is widely used in the field of, for example, clinical microorganism analysis, food safety test, and the like.

In MALDI-MS, a matrix solution is used for ionizing a target substance to be analyzed contained in a sample. A general matrix solution contains a component for dissolving a sample. Such a mixture solution of the matrix solution and the sample is placed and dried on a sample plate, and thus, a dried product of the mixture solution is obtained. The dried product is irradiated with laser to ionize the target substance contained in the dried product. The thus ionized target substance is detected as a peak of a mass spectrum. An analyzer can determine a microorganism based on a difference in the pattern of the peak.

In MALDI-MS for such microorganism determination, for example, a CHCA solution obtained by dissolving CHCA (α-cyano-4-hydroxycinnamic acid) in an organic solvent is used as the matrix solution. NPL 4 discloses a CHCA solution containing acetonitrile and ethanol, which are organic solvents, respectively in prescribed ratios.

### CITATION LIST

### NON PATENT LITERATURE

NPL 1: Victor Ryzhov and Catherine Fenselau, "Characterization of the Protein Subset Desorbed by MALDI from Whole Bacterial Cells", Anal. Chem., 2001, 73, 746-750.
NPL 2: Fernando J. Pineda et al., "Microorganism Identification by Matrix-Assisted Laser/Desorption Ionization Mass Spectrometry and Model-Derived Ribosomal Protein Biomarkers", Anal. Chem., 2003, 75, 3817-3822.
NPL 3: Teramoto K. et al., "Phylogenetic Classification of Pseudomonas putida Strains by MALDI-MS Using Ribosomal Subunit Proteins as Biomarkers", Anal. Chem., 2007, 79, 22, 8712-8719.
NPL 4: VITEK MS Matrix-CHCA Safety Data Sheet, bioMerieux Japan Ltd., June 3, 2021, [search date: May 12, 2022], Internet < URL: https://www.biomerieux.co.jp/sites/subsidiary_jp/files/SPS-revamp/411071_vitek_ms_matrix-chca_sds_04_bmxj.pdf>

### SUMMARY OF INVENTION

### TECHNICAL PROBLEM

In a method for determining a microorganism by MALDI-MS, it is preferable that a peak of a target substance is detected with high sensitivity. When a dried product formed using a conventional matrix solution is subjected to MALDI-MS, however, there has arisen a problem that sufficient sensitivity cannot be obtained particularly in a high mass region. Therefore, there has been a demand for means with which a peak of a target substance can be detected with high sensitivity in MALDI-MS.

The present disclosure has been devised to solve this problem, and objects thereof are to provide a matrix solution with which highly sensitive mass spectrum measurement can be performed in a matrix-assisted laser desorption/ionization mass spectrometry method, and to improve determination accuracy for a microorganism-derived sample.

### SOLUTION TO PROBLEM

A matrix solution according to a first aspect of the present disclosure is a matrix solution used to be mixed with a sample in a matrix-assisted laser desorption/ionization mass spectrometry method. The matrix solution is an aqueous solution containing acetonitrile, ethanol, trifluoroacetic acid, and water. A content of the acetonitrile is 30 to 40 vol% based on the matrix solution. A content of the ethanol is 10 to 20 vol% based on the matrix solution. A content of the trifluoroacetic acid is 1 to 3 vol% based on the matrix solution. The matrix solution contains α-cyano-4-hydroxycinnamic acid, 2,5-dihydroxybenzoic acid, or sinapinic acid in a ratio of 5 to 20 mg/ml.

A mass spectrometry method according to the first aspect of the present disclosure includes: mixing a sample and a matrix solution; drying the matrix solution having been mixed with the sample on a sample plate to form a dried product; and subjecting the dried product to mass spectrometry by a matrix-assisted laser desorption/ionization method to obtain a mass spectrum. The matrix solution is an aqueous solution containing acetonitrile, ethanol, trifluoroacetic acid, and water. A content of the acetonitrile is 30 to 40 vol% based on the matrix solution. A content of the ethanol is 10 to 20 vol% based on the matrix solution. A content of the trifluoroacetic acid is 1 to 3 vol% based on the matrix solution. The matrix solution contains α-cyano-4-hydroxycinnamic acid, 2,5-dihydroxybenzoic acid, or sinapinic acid in a ratio of 5 to 20 mg/ml.

### ADVANTAGEOUS EFFECTS OF INVENTION

According to the matrix solution of the present disclosure, a matrix solution with which highly sensitive mass spectrum measurement can be performed in a matrix-assisted laser desorption/ionization mass spectrometry method can be provided, and determination accuracy for a microorganism-derived sample can be improved.

### BRIEF DESCRIPTION OF DRAWINGS

Fig. 1 is a schematic diagram illustrating the configuration of an analysis device according to an embodiment.
Fig. 2 is a diagram illustrating compositions of matrix solvents.
Fig. 3 is a diagram for explaining a method for forming a dried product on a sample plate.
Fig. 4 is a diagram illustrating photographed images of dried products on a sample plate.
Fig. 5 is a diagram illustrating a mass spectrum of *E. coli.*
Fig. 6 is a diagram illustrating the numbers of detected ribosomal proteins.
Fig. 7 is a flowchart illustrating mass spectrometry processing and microorganism determination processing.
Fig. 8 is a diagram illustrating an experimental example of a mass spectrometry method of an embodiment.

### DESCRIPTION OF EMBODIMENTS

Now, one embodiment of the present disclosure (hereinafter referred to as the "present embodiment") will be described. It is noted that the present embodiment is not limited to the following. Herein, the expression in the form of "A to Z" means upper and lower limits of a range (namely, A or more and Z or less), and when A does not have a unit but only Z has a unit, the unit of A is the same as the unit of Z.

Moreover, herein, "%" means "vol%" unless otherwise stated.

The present embodiment will now be described in detail with reference to the accompanying drawings. It is noted that in the following description, the same or corresponding components are referred to with the same reference signs in the drawings to basically avoid redundant description.

### [1. Configuration of Analysis Device]

First, an example of an analysis device 1 for practicing a microorganism determination method of the present embodiment will be described. The microorganism determination method of the present embodiment includes a mass spectrometry method of the present embodiment.

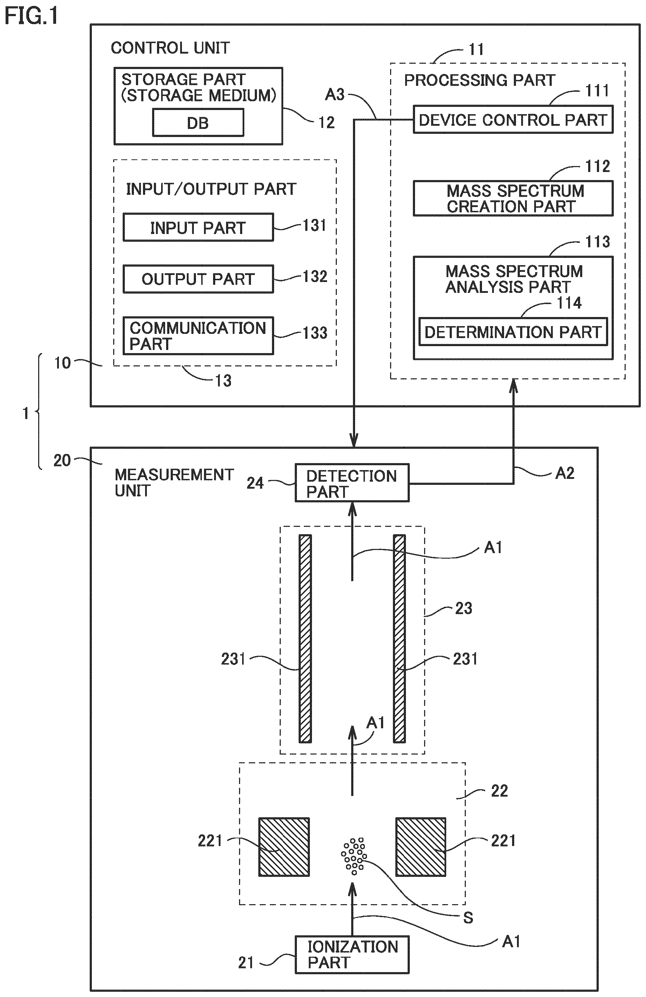

Fig. 1 is a schematic diagram illustrating the configuration of analysis device 1. Analysis device 1 is a mass spectrometer for performing mass spectrometry of a substance contained in a sample, and is, for example, MALDI-TOF MS (Matrix-Assisted Laser Desorption/Ionization Time-of-Flight Mass Spectrometry). Analysis device 1 determines the type of an organism by using a mass spectrum obtained by mass spectrometry.

The sample may be derived from a prokaryote, or may be derived from a eukaryote. The sample is preferably derived from a microorganism, and may contain an unknown microorganism. The prokaryote includes bacteria and archaea. Examples of the bacteria include bacteria belonging to the genus *Escherichia* (e.g., *E*. *coli*), the genus *Bacillus* (e.g., *Bacillus subtilis*), the genus *Lactobacillus* (e.g., lactic acid bacteria), the genus *Synechocystis* (e.g., cyanobacteria), and the genus *Mycobacterium* (e.g., actinomycetes). Examples of the archaea include the genus *Methanophilus,* the genus *Methanococcus,* the genus *Thermococcus,* and the genus *Phyllococcus.* The eukaryote includes animals, plants, fungi, and protists. The fungi include filamentous fungi, yeasts, mushrooms, molds, and the like, and encompass the phylum *Chytridiomycota,* the phylum *Zygomycota,* the phylum *Ascomycota,* the phylum *Basidiomycota,* the phylum *Glomeromycota,* the phylum *Microsporidia,* and the like. The phylum *Ascomycota* includes the genus *Aspergillus* (e.g., *Aspergillus oryzae*), the genus *Penicillium* (e.g., *Penicillium chrysogenum*), the genus *Saccharomyces* (e.g., budding yeast), and the like.

The sample contains a target substance, that is, a molecule to be analyzed. Moreover, in the present embodiment, the analysis with analysis device 1 includes detecting a peak of a mass spectrum, and measuring a mass-to-charge ratio (m/z) of a specific or nonspecific substance contained in the sample. In one example, the substance is a protein. The analysis with analysis device 1 includes determining, based on a m/z corresponding to a peak of the mass spectrum, the type of a microorganism from which the sample is derived. The m/z corresponding to a peak of a mass spectrum is generally referred to also as the "position" or "m/z position" of the peak.

Herein, the type of a microorganism includes at least one of taxonomic classes of the genotype, the strain, the subspecies, the species, the genus, and the family of a microorganism, unless otherwise stated. Moreover, the determination of the type of a microorganism includes classification, identification, and discrimination of the type of the microorganism. Hereinafter, the determination of the type of a microorganism is referred to simply as determination of the microorganism.

The analysis with analysis device 1 may include determining whether or not a specific substance is contained in the sample, and calculating the concentration of the specific substance in the sample.

Referring to Fig. 1, analysis device 1 includes a control unit 10 and a measurement unit 20.

Measurement unit 20 ionizes, with a high voltage, a substance (e.g., protein) contained in a sample, and detects the resultant ion S after separation in accordance with time of flight correlated with a m/z. Measurement unit 20 includes an ionization part 21, an ion acceleration part 22, a mass separation part 23, and a detection part 24. In Fig. 1, the movement of the ion S in measurement unit 20 is schematically illustrated with an arrow A1.

Ionization part 21 ionizes the substance contained in the sample by a matrix-assisted laser desorption/ionization (MALDI) method. As the ionization method, not only the MALDI method but also any soft ionization method such as an electrospray ionization (ESI) method can be employed. In the ionization performed by the ESI method, a configuration in which analysis device 1 further includes a liquid chromatograph for ionizing, with ionization part 21, a substance that is contained in the sample, and has been separated with the liquid chromatograph is preferred because high separability can be thus obtained.

Ionization part 21 includes a sample plate holder (not shown) for holding a sample plate, and an ion source including a laser device (not shown) for irradiating the sample plate with a laser beam. An analyzer first places a mixture solution of a sample and a matrix solution on the sample plate.

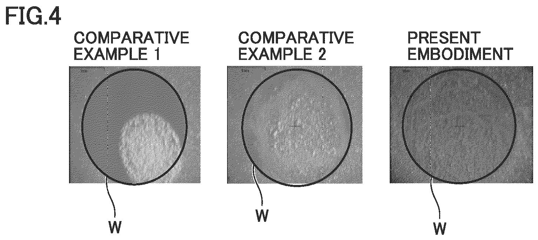

Specifically, for example, after precedently mixing a sample with a matrix solution, the analyzer drops the resultant matrix solution onto a well formed on the sample plate. The well is a circle drawn or engraved in the sample plate (see W illustrated in Fig. 4). On the other hand, a matrix solution may be mixed with a sample on the sample plate after being dropped onto the sample plate.

In the matrix solution, a matrix substance that easily absorbs a laser beam, and is easily ionized with the laser beam is contained. Herein, the matrix substance is also referred to simply as the "matrix". The matrix is used for analyzing a substance that is difficult to absorb a laser beam, and/or a substance that is easily damaged with a laser beam (such as a protein).

The sample to be mixed with the matrix solution may be in the form of a liquid, or a solid. More specifically, the sample may be, for example, a liquid containing a culture fluid of a bacterium, or may be a scrape, obtained with a toothpick or the like, of a colony of a bacterium having been cultured on a solid medium. The culture fluid of the bacterium contains the bacterium, and a liquid medium used for culturing the bacterium. The amount of the liquid medium contained in the sample to be mixed with the matrix solution is, however, preferably smaller. Therefore, preferably, the sample is centrifuged as a pretreatment, and a portion (precipitate) of the sample remaining after removing a supernatant is mixed with the matrix solution. More preferably, the precipitate is further centrifugally washed with ultrapure water or the like, and the resultant is then mixed with the matrix solution.

The sample may be a purified product of a cell component such as a ribosomal protein instead of the cell itself of a microorganism as described above.

A mixing ratio between the matrix solution and the sample is not limited as long as the effect of the present invention is exhibited, and for example, the sample is mixed in an amount, in terms of a volume ratio, equal to or less than a fraction of, and equal to or more than one hundredth of the matrix solution.

The analyzer obtains a dried product by drying, on the sample plate, the matrix solution with which the sample has been mixed as described above. Thereafter, the sample plate is set on the sample plate holder disposed in a vacuum container of ionization part 21.

It is noted that the dried product of the mixture solution of the sample and the matrix solution is generally referred to as the "crystal" in some cases, and more specifically, is variously referred to as a "crystal", "mixed crystal", "sample crystal", "matrix crystal", "sample/matrix crystal", and the like.

In the following description, the matrix solution having been placed on the sample plate to be formed into the dried product is described as including the matrix solution with which the sample has been mixed as described above (namely, the "mixture solution"), unless otherwise stated.

Ionization part 21 depressurizes the vacuum container in which the sample plate has been set, and then irradiates the dried product on the sample plate with a laser beam for ionization of the target substance contained in the dried product. The type of the laser device for emitting the laser beam is not especially limited as long as it can oscillate light absorbed by the used matrix solution, and for example, when the matrix solution contains CHCA, N2 laser (wavelength of 337 nm) or the like can be suitably used. An ion S of the target substance having been ionized by ionization part 21 is extracted from an electric field formed by an extraction electrode or the like not shown, and is introduced into ion acceleration part 22.

Ion acceleration part 22 includes an accelerating electrode 221, and accelerates the ion S having been introduced thereinto. The flow of the accelerated ion S is appropriately converged by an ion lens, which are not shown, to be introduced into mass separation part 23.

Mass separation part 23 includes a flight tube 231, and separates ions S in accordance with a difference in time of flight spent by the respective ions S flying inside flight tube 231. Although Fig. 1 illustrates linear flight tube 231, a reflectron flight tube, a multi-turn flight tube or the like may be used. The method of mass spectrometry is not especially limited as long as ions S contained in the sample can be separated and detected.

Detection part 24 includes an ion detector such as a multi-channel plate, detects the ion S separated by mass separation part 23, and outputs a detected signal with an intensity according to the number of ions having entered detection part 24. The detected signal output from detection part 24 is input to a processing part 11 of control unit 10. In Fig. 1, a flow of the detected signal of the ions S from detection part 24 of measurement unit 20 is schematically illustrated with an arrow A2.

Control unit 10 includes processing part 11, a storage part 12, and an input/output part 13.

Processing part 11 is configured by including a processor such as a CPU, and functions as a main part in an operation for controlling analysis device 1. Processing part 11 performs various processing by executing a program stored in storage part 12 and the like.

Processing part 11 includes a device control part 111, a mass spectrum creation part 112, and a mass spectrum analysis part 113. Mass spectrum analysis part 113 includes a determination part 114.

Device control part 111 controls the operation of measurement unit 20 based on data related to analysis conditions input from an input part 131 described below. In Fig. 1, the control of measurement unit 20 by device control part 111 is schematically illustrated with an arrow A3.

Mass spectrum creation part 112 converts the time of flight into a m/z based on measurement data including the amount of ions detected by detection part 24, and the time of flight of the ions, and creates a mass spectrum indicating a detection amount corresponding to each m/z.

The number of detected signals of the target substance detected by detection part 24, and the intensities of the detected signals correlate with the number of peaks corresponding to the target substance in the mass spectrum, and the intensities of the peaks. Moreover, a ratio of the intensity of a detected signal of the target substance to the intensity of a noise detected by detection part 24 (S/N ratio of the detected signal) correlates with a ratio of the intensity of a peak of the target substance to the intensity of a noise in the mass spectrum (S/N ratio in the mass spectrum). Therefore, herein, a value correlated with at least one of the number of peaks corresponding to a target substance, and the intensities of the peaks in the mass spectrum, and the ratio of the intensity of the peak to the intensity of a noise (S/N ratio) in the mass spectrum is referred to as the "sensitivity". In addition, at least one of a state in which the number of peaks corresponding to the target substance is large, a state in which the intensities of the peaks are high, and a state in which the S/N ratio of the mass spectrum is high is referred to as the "high sensitivity".

Mass spectrum analysis part 113 detects, in the mass spectrum, a peak of the mass spectrum. It calculates a m/z corresponding to the detected peak. Mass spectrum analysis part 113 may determine, based on protein database or the like, a substance corresponding to a m/z indicated by the peak of the mass spectrum. In other words, mass spectrum analysis part 113 can calculate a m/z of a specific or nonspecific substance contained in the sample. Mass spectrum analysis part 113 may further determine, based on the m/z, whether or not the specific substance is contained in the sample (component identification in the sample).

Mass spectrum analysis part 113 includes determination part 114. In one example, determination part 114 creates database including a mass spectrum. The database includes one or more, and preferably a large number of mass spectra of microorganisms of known types. Determination part 114 determines the type of a microorganism using the database of mass spectra.

In one example, determination part 114 determines a microorganism by a fingerprint method. Specifically, determination part 114 determines a microorganism by comparing the pattern of a mass spectrum of an unknown microorganism with the pattern of a mass spectrum of a known microorganism stored in the database. The determination of a microorganism is performed, for example, by referring to a peak of a biomarker that is a substance showing an expression pattern characteristic to each microorganism. The determination of a microorganism based on the pattern of a peak of a biomarker will be described in detail below.

Storage part 12 includes a nonvolatile storage medium. Storage part 12 stores the mass spectrum created by mass spectrum creation part 112, the measurement data output from measurement unit 20, the program used for executing processing by processing part 11, and the like. Storage part 12 corresponds to an example of a "memory" of the present disclosure. Storage part 12 may include a storage medium removable from analysis device 1. The storage medium may be any medium capable of storing various data, such as a CD (compact disc), a DVD (digital versatile disc), and a USB (universal serial bus) memory. Storage part 12 stores the database of mass spectra.

Input/output part 13 is an interface for inputting/outputting information between analysis device 1 and the outside. Input/output part 13 includes an input part 131, an output part 132, and a communication part 133.

Input part 131 is configured by including an input device such as a mouse, a keyboard, various buttons and/or a touch panel. Input part 131 receives, from an analyzer, information necessary for control of the operation of measurement unit 20, and information necessary for processing performed by processing part 11, and the like.

Output part 132 is configured by including a display device such as a liquid crystal monitor, a printer, and the like. Output part 132 displays, in a display device, information on the measurement by measurement unit 20, and results of the processing by processing part 11, or prints these on print media.

Communication part 133 is configured by including a communication device capable of communication through wireless or wired connection such as Internet. Communication part 133 receives data necessary for the processing by processing part 11, transmits data having been processed by processing part 11, such as determination results, and appropriately receives/transmits necessary data.

A part or the whole of the function of control unit 10 described above may be disposed in a computer, a server, or the like physically separated from measurement unit 20. Moreover, control unit 10 may be configured by a plurality of computers. For example, it may be configured by a first computer for creating the database of mass spectra, and a second computer for determining a microorganism by using the database of mass spectra as described below. In this case, for example, the first computer creates the database of mass spectra based on detected signals of the ions S output from measurement unit 20, and stores it in the storage medium. The second computer obtains the database of mass spectra from the storage medium, and determines the type of a microorganism by using the database. For transfer of the database from the first computer to the second computer, a wired or wireless network may be used.

Analysis device 1 used in the present embodiment is not especially limited as long as it is combined with a MALDI (matrix-assisted laser desorption/ionization) ion source. It may be, for example, a MALDI-IT (matrix-assisted laser desorption/ionization-ion trap) mass spectrometer, a MALDI-IT-TOF (matrix-assisted laser desorption/ionization-ion trap-time of flight) mass spectrometer, or a MALDI-FTICR (matrix-assisted laser desorption/ionization-Fourier transform ion cyclotron resonance) mass spectrometer. Moreover, analysis conditions employed in analysis device 1 are set within a range usually set by those skilled in the art.

### [2. Conventional Mass Spectrometry Method]

Conventionally, there is a method for determining a microorganism by mass spectrometry. In mass spectrometry, a mass spectrum corresponding to an analysis result can be obtained easily and in a short period of time by using a very small amount of a microorganism sample. Moreover, analysis of multiple samples can be performed rapidly and easily by employing automatic analysis.

In such mass spectrometry, in particular, analysis of a microorganism by MALDI-MS, that is, one of soft ionization methods for ionizing a biopolymer such as a protein substantially without degrading it, is widely utilized.

In the analysis of a microorganism by MALDI-MS, a matrix solution having been mixed with a sample is dried on a sample plate to vaporize a solvent contained in the matrix solution, and thus, a dried product containing a target substance is formed. When the dried product on the sample plate is irradiated with a laser beam, a matrix substance absorbs the energy of the laser beam to be heated, and vaporize. At this point, the target substance also vaporizes together with the matrix substance, and in this process, the target substance is ionized.

As the sample plate used for the formation of a dried product and the mass spectrometry, a disposable plate suitable for application to clinical microorganism analysis and food safety test is used in many cases. In the field of microorganism study, however, a sample plate suitable for repeated use is used in some cases. The sample plate contains, for example, stainless steel or a conductive resin.

As the matrix, from the viewpoint of efficiently ionizing a protein sample, for example, CHCA, sinapinic acid, 2,5-dihydroxybenzoic acid and the like are used. In general, the matrix is dissolved in a solvent to obtain a matrix solution, which is mixed with a sample.

Various compositions of the matrix solution have been proposed. For example, NPLs 1 to 4 disclose compositions of various matrix solvents for CHCA. Table 1 shows the compositions of the matrix solvents, disclosed in NPLs 1 to 4, containing substances, such as acetonitrile (ACN), ethanol (EtOH), water, and trifluoroacetic acid (TFA), in prescribed contents. These solvents are used to prepare matrix solutions containing 5 to 15 mg/mL of CHCA.

### [Table 1]

**[Table 1]**

| | NPL 1 | NPL 2 | NPL 3 | NPL 4 |
|---|---|---|---|---|
| Composition of Matrix Solvent | ACN 70% | ACN 70% | ACN 50% | ACN 20% or more |
| | TFA 0.03% | TFA 1.5% | TFA 1% | and less than 30% |
| | water 29.7% | water 28.4% | water 49% | EtOH 20% or more and less than 30% |
| | | | | TFA 3% or more and less than 5% |
| | | | | others 33% or more and less than 45% |

When a dried product obtained by using such a matrix solution is subjected to MALDI-MS, however, there has arisen a problem that sufficient sensitivity cannot be obtained particularly in a high mass region. It is noted that the high mass region refers to a region in which a m/z is comparatively high, and is, for example, a region having a m/z of 10,000 or more, and more restrictively a region having a m/z of 11,000 or more.

In determination of a microorganism, it is important to obtain sufficient sensitivity. In particular, it is important to obtain sufficient sensitivity in the high mass region. This is because a protein having a higher molecular weight has a longer amino acid sequence, and hence there is a higher possibility of occurrence of mutation. Therefore, in determination of a microorganism with high affinity, it is important to detect a peak in the high mass region. The determination of a microorganism with high affinity refers to, for example, determination of a subspecies or a strain of the same species.

Therefore, the present inventors have examined experimental conditions necessary for obtaining sufficient sensitivity also in the high mass region.

### [3. Examination of Process for Forming Dried Product Using Conventional Matrix Solution]

In the examination of experimental conditions, the present inventors have presumed that there is a problem in the process for forming a dried product in the conventional mass spectrometry method, and have searched for the solution.

First, the present inventors have paid attention to that in the process for forming a dried product by dropping the conventional matrix solution onto a well formed on a sample plate, the matrix solution is shifted in a narrow region in many cases. In such a case, when a sample crystal is formed at an end of the well, it may not be suitably irradiated with laser in automatic measurement in some cases. In such a case, there is a possibility that a mass spectrum resulting from the analysis of the sample may not have sufficient sensitivity.

Next, in order to inhibit a dried product from being formed locally at an end of the well, the present inventors have examined that the matrix solution increased in the amount is dropped to form a dried product in the entire well. When the amount of the matrix solution to be dropped onto the well is increased, however, there is a possibility that time required for drying the matrix solution becomes so long, and/or the resultant dried product is so thick that a favorable mass spectrum cannot be obtained.

Accordingly, the present inventors have examined that a dried product is to be formed in an entire well without increasing the amount of the matrix solution to be dropped onto the well by considering the composition of the matrix solution.

### [4. Examination of Preferable Composition of Matrix Solution]

First, the present inventors have examined reduction of the surface tension by adding a large amount of an organic solvent for spreading the matrix solution over the entire well. When the concentration of the organic solvent in the matrix solution is too high, however, a large amount of a lipid elutes from a bacterial cell, and there is a high possibility that the ionization of the protein may be inhibited. Moreover, when the concentration of the organic solvent in the matrix solution is too high, since a drying speed is increased, a small dried product not suitable for ionization of the protein is formed, and hence it is presumed that a peak intensity in a mass spectrum may be reduced. Therefore, it is presumed that it is necessary to work out a composition of a matrix reagent suitable for MALDI-MS measurement of a cell component of a microorganism principally of a protein not by simply increasing the organic solvent but by another method.

Based on the results of these examinations, the present inventors have found that a mass spectrum with high sensitivity also in the high mass region can be obtained by forming a dried product satisfying the following first to third conditions.

The first condition is that a dried product is formed widely, and more preferably over an entire well. Herein, that a dried product is formed widely refers to that the dried product is formed to have a large area on the upper face thereof (face to be irradiated with laser). When the dried product is formed widely, and preferably over an entire well, a detected signal of a target substance is obtained from the wide area in mass spectrometry. Accordingly, the sensitivity is directly increased.

The second condition is that the dried product has high uniformity. An example of high uniformity of the dried product is high uniformity of the shape on the upper face of the dried product. As an example of high uniformity of the shape on the upper face of the dried product, for example, change, among positions, in the thickness in the vertical direction of the dried product is small, or, for example, the upper face of the dried product is less irregular and is almost flat.

Another example of the high uniformity of the dried product is high uniformity of the internal structure of the dried product. The high uniformity of the internal structure of the dried product is, for example, a ratio and/or a binding form between the target substance and the matrix in the dried product is less different among positions at least on the upper face of the dried product. Alternatively, for example, the dried product in the well is united without forming a plurality of small dried products.

When the uniformity of the dried product is high, the protein is suitably ionized to increase the signal intensity, and thus, the sensitivity is improved.

The third condition is that a dried product is derived from a mixture solution in which a bacterial component of a microorganism is favorably extracted. The favorable extraction of a bacterial component of a microorganism refers to, for example, that a bacterial component of the microorganism is highly efficiently extracted, and more specifically, that the extraction amount of the target substance per microorganism is large. Thus, the concentration of the target substance in the dried product is increased to obtain a detected signal of the target substance strongly, and hence, the sensitivity is improved.

In other words, the present inventors have presumed that a mass spectrum with high sensitivity can be obtained when a matrix solution that forms a dried product satisfying the first to third conditions can be obtained. Moreover, the present inventors have considered that sufficient sensitivity can be obtained also in the high mass region, in which sufficient sensitivity could not be conventionally obtained, as a result of the sensitivity improved in the entire mass spectrum.

Based on the results of the observation and studies described so far, the present inventors have invented a composition of a matrix solution that forms a dried product with which a mass spectrum with high sensitivity can be obtained.

The present inventors have found that a dried product with which a peak can be detected with high sensitivity in the high mass region can be obtained by particularly considering the composition of a matrix solvent. Herein, the composition of a matrix solvent encompasses the types of a reagent and a substance contained in the matrix solvent, and contents and/or concentrations of the reagent and the substance.

### [5. Mass Spectrometry Using Matrix Solution of Embodiment]

### (5-1. Composition of Matrix Solution of Embodiment)

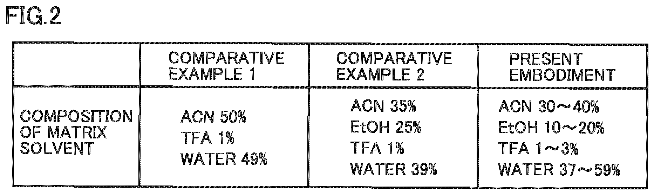

Fig. 2 is a diagram illustrating compositions of matrix solvents of Comparative Examples and the present embodiment. In Fig. 2, volume ratios of acetonitrile, ethanol, trifluoroacetic acid, and water in each matrix solvent are shown as percentages obtained by assuming that the volume of the entire matrix solvent is 100. Each of acetonitrile, ethanol, trifluoroacetic acid, and water described above may be produced respectively by known methods, or commercially available products may be obtained.

Referring to Fig. 2, the matrix solvent of the present embodiment is characterized by that the ratios of acetonitrile and ethanol, that is, organic solvents, are different from those in the matrix solvents of Comparative Examples 1 and 2.

Specific compositions will now be described. The organic solvent contained in the matrix solvent of Comparative Example 1 is 50% of acetonitrile, and the organic solvent contained in the matrix solvent of Comparative Example 2 is 35% of acetonitrile and 25% of ethanol.

On the other hand, the organic solvent contained in the matrix solvent of the present embodiment is 30 to 40% of acetonitrile and 10 to 20% of ethanol, more preferably 33 to 37% of acetonitrile and 13 to 17% of ethanol, and further preferably about 35% of acetonitrile and about 15% of ethanol. The matrix solvent of the present embodiment further contains 1 to 3% of trifluoroacetic acid, and more preferably 1 to 2% trifluoroacetic acid. It goes without saying that the balance of the matrix solvent is water.

In one aspect of the present embodiment, the content of the acetonitrile contained in the matrix solvent is, in terms of a volume ratio, 30 to 40%, preferably 33 to 37%, and more preferably about 35%.

In one aspect of the present embodiment, the content of the ethanol contained in the matrix solvent is, in terms of a volume ratio, 10 to 20%, preferably 13 to 17%, and more preferably about 15%.

Each matrix solvent is prepared so that the total ratio of acetonitrile, ethanol, trifluoracetic acid, and water can be 100%. The preparation method is not especially limited, and for example, it may be prepared by adding, in a test tube, acetonitrile, ethanol, and trifluoroacetic acid to water, or may be produced on an industrial scale in a chemical plant or the like by adding acetonitrile, ethanol, and trifluoroacetic acid to water.

The matrix solvent of the present embodiment is prepared to further contain a matrix in an amount of 5 to 20 mg/ml, preferably 5 to 15 mg, and further preferably 8 to 10 mg/ml, and the resultant is used as a matrix solution.

In one example, the matrix is α-cyano-4-hydroxycinnamic acid (4-CHCA, herein sometimes referred to simply as CHCA), that is, one of matrices most frequently used in mass spectrum. The matrix may be 2,5-dihydroxybenzoic acid, or sinapinic acid, that is, other matrices similarly most frequently used in mass spectrum. Other examples of the matrix may include, but are not limited to, 3-hydroxy-4-nitrobenzoic acid (3H4NBA), α-cyano-3-hydroxycinnamic acid (3-CHCA), ferulic acid, or 1,5-diaminonaphthalene.

In Figs. 4 to 6, the experimental result obtained using, as an example of the matrix solution of the present embodiment, a solution containing 10 mg/mL of CHCA in a matrix solvent containing 35% of acetonitrile, 15% of ethanol, 1% of trifluoroacetic acid, and 39% of water is illustrated as an example. As an example of the matrix solution of Comparative Example 1, a solution containing 10 mg/mL of CHCA in a matrix solvent containing 50% of acetonitrile, 1% of trifluoroacetic acid, and 49% of water is used. As an example of the matrix solution of Comparative Example 2, a solution containing 10 mg/mL of CHCA in a matrix solvent containing 35% of acetonitrile, 25% of ethanol, 1% of trifluoroacetic acid, and 39% of water is used.

### (5-2. Formation of Dried Product Using Matrix Solution of Embodiment)

A dried product is formed by mixing the matrix solution of the present embodiment with a sample, and drying the resultant on a sample plate.

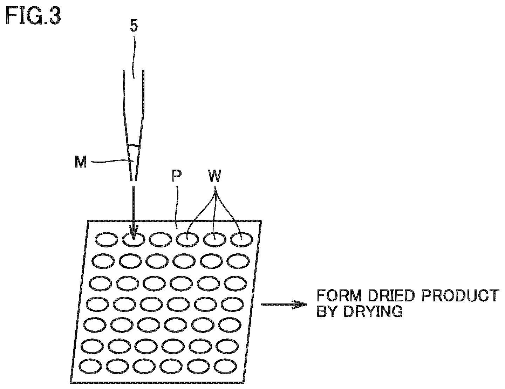

Fig. 3 is a diagram for explaining a method for forming a dried product on a sample plate. Fig. 3 illustrates a state in which a matrix solution M is disposed with a pipette 5 on a well W of a sample plate P.

Sample plate P is a general plate for MALDI, and is, for example, a plate containing stainless steel and/or a conductive resin.

The sample is a sample derived from a microorganism, and in one example, is a sample derived from *E. coli.* An analyzer adds 9 µL of a matrix solution to a microtube, and further adds 1 µL of a culture fluid of *E. coli* thereto, well mixes the resultant, and drops 1 µL of the resultant mixture solution onto sample plate P.

A dried product is formed by drying the matrix solution thus disposed on well W of sample plate P.

According to the observation performed by the present inventors with the naked eye and a camera device, a dried product formed from the matrix solution of Comparative Example 1 (hereinafter also referred to as the dried product of Comparative Example 1), and a dried product formed from the matrix solution of Comparative Example 2 (hereinafter also referred to as the dried product of Comparative Example 2) are formed to be shifted in a narrow region on the plate in many cases. In relation, the dried product is formed at an end of the plate in some cases. On the other hand, a dried product formed from the matrix solution of the present embodiment (hereinafter also referred to as the dried product of the present embodiment) is uniformly spread on the entire plate. Fig. 4 illustrates examples of the outlines of the dried product of Comparative Example 1, the dried product of Comparative Example 2, and the dried product of the present embodiment.

Fig. 4 is a diagram illustrating photographed images of the dried products on the sample plate. Fig. 4 illustrates black and white images of the dried products photographed with a camera including, as an image sensor, a CCD (charge coupled device) of ionization part 21 of analysis device 1 (CCD camera).

As illustrated in Fig. 4, the dried product of Comparative Example 1 is formed to be shifted to a lower right portion of well W. The dried product is bright, and has dense particulate irregularities (not shown). Moreover, a part of the dried product is overflowed out of the outer frame of well W.

The dried product of Comparative Example 2 is rather bright in the center, and has particulate irregularities (not shown). The outer edge of the dried product of Comparative Example 2 is darker than in the center thereof, and appears to spread flatly.

The dried product of the present embodiment uniformly spreads widely in the entire well, and hence is thin as a whole, and is so dark that it is difficult to distinguish from the base of the sample plate in the CCD black and white image.

In this manner, the dried product of the present embodiment is in a state different from those of the dried product of Comparative Example 1 and the dried product of Comparative Example 2. The difference in the state of the dried products probably reflects change of at least one of the strength of surface tension, the drying speed, a difference in the type and/or the amount of a bacterial component contained in the dried product, and a difference in the uniformity of the dried product caused by changing the concentrations of acetonitrile and ethanol.

More specifically, the dried product of the present embodiment is wide and has a uniform thickness as compared with the dried product of Comparative Example 1 and the dried product of Comparative Example 2. The present inventors have confirmed that this characteristic of the dried product of the present embodiment does not depend on samples but has high reproducibility.

In relation, the present inventors have confirmed that the matrix solution of the present embodiment has a higher drying speed than the matrix solutions of Comparative Examples 1 and 2, and forms a dried product in a shorter period of time. This high drying speed probably reflects mainly a difference in the vaporization speed due to the composition (in particular, the concentrations of acetonitrile and ethanol) itself, and a difference in the surface area in contact with the air of the matrix solution having been dropped onto the sample plate (in particular, a difference in the area of the upper face of the matrix solution). There is a possibility that the difference in the drying speed may reflect at least one of a difference in the amount and/or the type of the bacterial component contained in the mixture solution, and the uniformity of the mixture solution (the ratios, or uniformity of binding form between the target substance and the matrix). Owing to this high drying speed, a dried product having high uniformity described above can be formed in a shorter period of time.

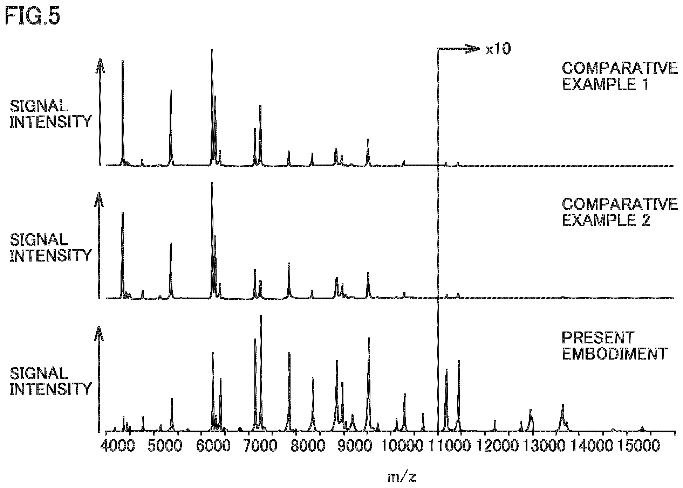

It is presumed that such a characteristic crystalline state of the dried product of the present embodiment is reflected to obtain a mass spectrum with high sensitivity as illustrated in subsequent Fig. 5.

### (5-3. Result of Mass Spectrometry with Dried Product Using Matrix Solution of Embodiment)

Fig. 5 is a diagram illustrating mass spectra of *E. coli,* and includes, in the downward direction, a mass spectrum of the dried product of Comparative Example 1 (hereinafter also referred to as the "mass spectrum of Comparative Example 1"), a mass spectrum of the dried product of Comparative Example 2 (hereinafter also referred to as the "mass spectrum of Comparative Example 2"), and a mass spectrum of the dried product of the present embodiment (hereinafter also referred to as the "mass spectrum of the present embodiment"). In Fig. 5, the abscissa indicates the m/z, and the ordinate indicates the detected signal intensity. In a region of a m/z of 11,000 or more, 10-fold signal intensity is shown.

Referring to Fig. 5, the mass spectrum of the present embodiment is improved, as compared with the mass spectra of Comparative Example 1 and Comparative Example 2, in the intensities of peaks as a whole, and is improved in the sensitivity. In particular, in the high mass region (for example, a region of a m/z of about 11,000 or more), substantially no peak is detected in each of the mass spectra of Comparative Example 1 and Comparative Example 2, and a very small number of detected peaks have very low intensity, but in the mass spectrum of the present embodiment, a large number of peaks with high intensity are detected. In this manner, the mass spectrum of the present embodiment is improved in the sensitivity as a whole, and is improved in the sensitivity remarkably in the high mass region in particular. As a result, sufficient sensitivity can be obtained also in the high mass region. In other words, when the matrix solution of the present embodiment is used, a peak can be detected with high sensitivity.

Moreover, since a large number of peaks with high intensities are detected in the mass spectrum of the present embodiment, it is understood that extraction itself of a bacterial component from the microorganism can be favorably performed. In other words, the matrix solution of the present embodiment can extract the bacterial component highly efficiently, with at least equivalent or higher efficiency to or than the matrix solutions of Comparative Examples 1 and 2.

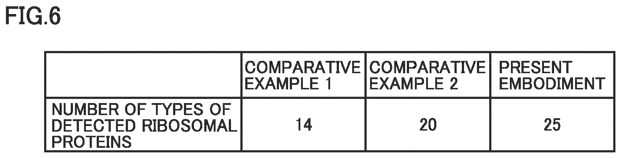

Fig. 6 is a diagram illustrating the number of detected ribosomal proteins.

More specifically, Fig. 6 is a peak list of respective peaks in mass spectra as those partially illustrated in Fig. 5 including corresponding m/zs and peak intensities. Fig. 6 illustrates the number of ribosomal proteins contained in the peak list calculated by comparing, with a m/z of the peak list, a theoretical m/z calculated based on the molecular weight of a ribosomal protein known to be expressed in *E. coli.*

In the dried product obtained using the matrix solution of the present embodiment, 25 types of ribosomal proteins on average can be detected in 16 samples. On the other hand, in the dried products obtained using the matrix solutions of Comparative Examples 1 and 2, 14 types and 20 types are respectively detected. In other words, in the dried product of the present embodiment, a larger number of ribosomal proteins can be detected than in Comparative Examples 1 and 2. Specifically, when the matrix solution of the present embodiment is used, a microorganism can be determined by using a larger number of biomarkers as compared with a case in which the matrix solutions of Comparative Examples 1 and 2 are used.

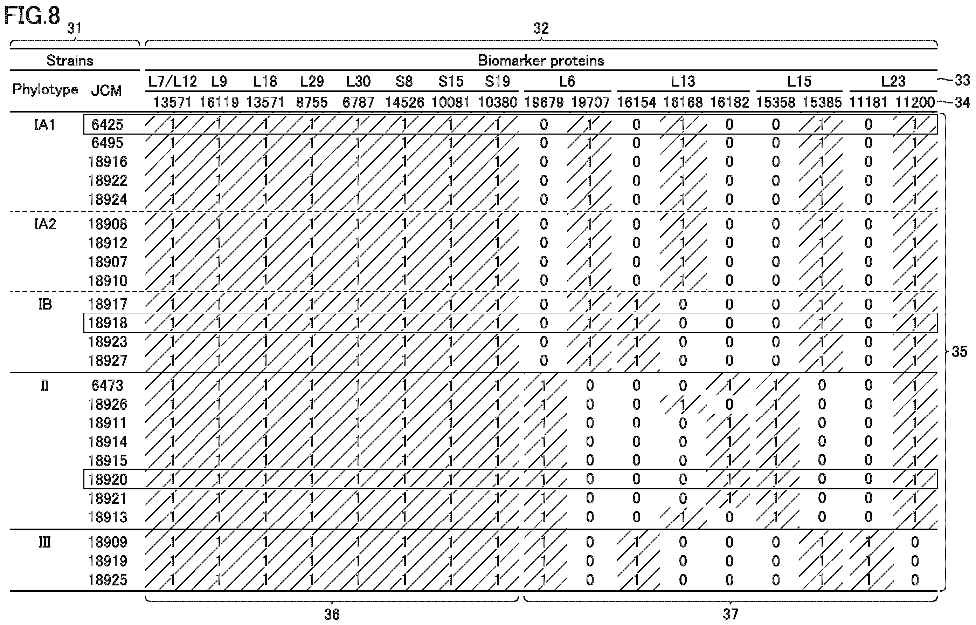

Referring to Figs. 5 and 6, when the matrix solution of the present embodiment is used, the sensitivity is improved in the entire mass spectrum, and in particular, sufficient sensitivity can be obtained also in the high mass region. In this manner, accuracy of determining a microorganism based on the mass spectrum is improved. As a result, a microorganism having a high degree of affinity can be classified as illustrated in Fig. 8 described below.

### (5-4. Effects of Matrix Solution and Mass Spectrometry Method of Embodiment)

As illustrated in Fig. 4, when the matrix solution of the present embodiment is used, a dried product widely and uniformly spread in a well can be prepared. As a result, since uniform signals can be obtained widely from the upper face of the dried product, a mass spectrum with high sensitivity can be highly efficiently obtained.

In relation, since a mixture solution is widely spread in a well, the drying speed is improved. Therefore, a homogeneous dried product can be prepared in a short period of time, and hence a favorable sample can be efficiently prepared.

Since a bacterial component can be favorably extracted, the number of proteins derived from a microorganism that can be detected by MALDI-MS is increased. In other words, the number of peaks detected in a mass spectrum is increased. Such increase of the amount of information also makes a contribution to improvement of accuracy of determining a microorganism.

Owing to these effects, the accuracy of determining a microorganism by the mass spectrometry method using the matrix solution of the present embodiment is improved.

### [6. Mass Spectrometry Processing and Microorganism Determination Processing]

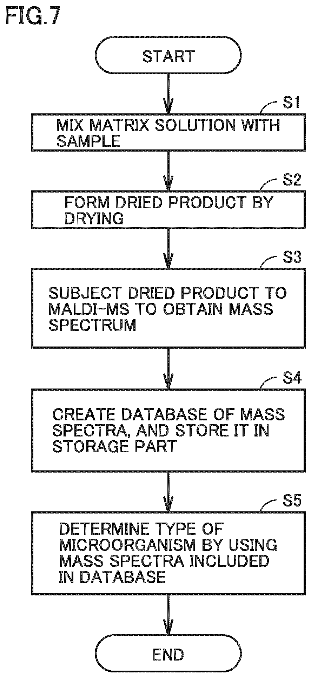

Fig. 7 is a flowchart illustrating mass spectrometry processing and microorganism determination processing. Steps illustrated in Fig. 7 are performed manually by an analyzer with general experimental devices used in culture and mass spectrometry of a microorganism. In the drawing, "S" is used as an abbreviation of "STEP".

In S1, an analyzer mixes a matrix solution of the present embodiment with a sample.

In S2, the analyzer dries the matrix solution having been mixed with the sample on a sample plate to form a dried product.

In S3, the analyzer performs mass spectrometry by matrix-assisted laser desorption/ionization on the dried product to obtain a mass spectrum.

In S4, the analyzer creates database including mass spectra, and stores it in storage part 12.

In S5, the analyzer determines the type of a microorganism by using the mass spectra included in the database.

According to the processing illustrated in Fig. 7, by using the matrix solution according to the present embodiment, a mass spectrum with high sensitivity can be obtained, and a large number of peaks having sufficient intensities can be detected particularly in the high mass region. As a result, accuracy of determining a microorganism can be improved.

In the aforementioned example, it is shown that a mass spectrum with high sensitivity can be measured in MALDI-MS by using the matrix solution of the present embodiment, and as a result, the determination accuracy for a microorganism-derived sample is improved, and the matrix solution of the present embodiment is obviously applicable to a biological sample derived from an organism other than a microorganism.

It is noted that the type, the content, and/or the concentration of a substance contained in the matrix solution of the present embodiment are described merely as examples. In particular, the type, the content, and/or the concentration of a substance except for acetonitrile and ethanol (e.g., trifluoroacetic acid, water, and a matrix) can be changed within a range usually considered and adjustable by those skilled in the art, and also in such a case, the effects equivalent to those of the above-described embodiment will be probably obtained. For example, a matrix solution containing a plurality of matrices adjusted to a total concentration of 8 to 20 mg/ml may be used as long as the effects of the present application are obtained. The type, the content, and/or the concentration of a substance contained in the matrix solution of the present embodiment (e.g., acetonitrile, or ethanol) can be measured with, for example, a gas chromatograph.

### [7. Examples]

Now, results of microorganism analysis performed by a mass spectrometry method using the matrix solution of the present embodiment will be described.

Fig. 8 is a table showing peaks of ribosomal proteins obtained as a result of mass spectrometry of *Cutibacterium acnes* (*C. acnes*) by the mass spectrometry method using the matrix solution of the present embodiment.

A left column 31 of Fig. 8 shows strains of C. *acnes,* with respect to phylotypes, by using management Nos. used in Japan Collection of Microorganisms (JCM) of Institute of Physical and Chemical Research.

A right column 32 shows detection results of proteins serving as biomarkers (biomarker proteins). A row 33 shows names of ribosomal proteins corresponding to biomarkers. A row 34 below row 33 shows the molecular weights of the respective ribosomal proteins. A row 35 shows, with respect to each strain, whether or not a peak is detected at the molecular weight by using 1 (detected) or 0 (not detected).

In ribosomal proteins of L7/L12 to S19 shown in a region 36, the molecular weight of a specific ribosomal protein did not change depending on the strain. Therefore, even when peaks of L7/L12 to S19 were used, the strain of *C*. *acnes* could not be determined.

On the other hand, in ribosomal proteins of L6, L13, L15 and L23 shown in a region 37, the molecular weight was different depending on the strain. More specifically, in the ribosomal proteins of L6, L13, L15, and L23, the molecular weight was different by about 10 to 30 depending on the strain. Therefore, it was found that the strain of *C*. *acnes* can be determined based on peaks of L6, L13, L15, and L23. For example, three strains of reference Nos. 6425, 18918, and 18920 could be determined based on the peak of L13. In this manner, it was found that L6, L13, L15, and L23 could be used as biomarkers for determining a strain of C. *acnes.*

Variations of the molecular weight of the ribosomal proteins of L6, L13, L15, and L23 are respectively 19679/19707, 16154/16168/16182, 15358/15385, and 11181/11200, all of which are included in the high mass region of the molecular weight of 11,000 or more. Therefore, a strain of C. *acnes* could be determined based on the high mass ribosomal proteins.

As described above, a microorganism could be determined in C. *acnes* by obtaining a peak in the high mass region. Moreover, it was found that it is important to detect a peak in the high mass region for determining a strain in C. *acnes.*

When sufficient sensitivity is obtained also in the high mass region in this manner, the accuracy of determining a microorganism is improved. As a result, even microorganisms having similar genetic information and having high affinity, which are comparatively difficult to determine, can be determined. Accordingly, in determination of a microorganism, particularly in determination of a microorganism with high affinity, it is important to obtain sufficient sensitivity also in the high mass region.

### [Aspects]

Those skilled in the art will understand that the above-described plurality of exemplifying embodiments are specific examples of the following aspects.

(Item 1) A matrix solution according to one aspect is a matrix solution used to be mixed with a sample in a matrix-assisted laser desorption/ionization mass spectrometry method. The matrix solution is an aqueous solution containing acetonitrile, ethanol, trifluoroacetic acid, and water. A content of the acetonitrile is 30 to 40 vol% based on the matrix solution. A content of the ethanol is 10 to 20 vol% based on the matrix solution. A content of the trifluoroacetic acid is 1 to 3 vol% based on the matrix solution. The matrix solution contains α-cyano-4-hydroxycinnamic acid, 2,5-dihydroxybenzoic acid, or sinapinic acid in a ratio of 5 to 20 mg/ml.

According to the matrix solution of item 1, a matrix solution capable of measuring a mass spectrum with high sensitivity by MALDI-MS can be provided. Moreover, when the sample is a sample derived from a microorganism, accuracy of determining a microorganism using a mass spectrum can be improved.

(Item 2) In the matrix solution according to item 1, the content of the acetonitrile is more preferably 33 to 37 vol% based on the matrix solution. The content of the ethanol is more preferably 13 to 17 vol% based on the matrix solution.

(Item 3) The matrix solution according to item 1 or 2 more preferably contains α-cyano-4-hydroxycinnamic acid in a ratio of 8 to 10 mg/ml.

(Item 4) In the matrix solution according to any one of items 1 to 3, the sample is a sample derived from a microorganism.

When the sample is a sample derived from a microorganism as described in item 4, the accuracy of determining a microorganism using a mass spectrum can be improved.

(Item 5) A mass spectrometry method according to one aspect includes: mixing a sample and a matrix solution; drying the matrix solution having been mixed with the sample on a sample plate to form a dried product; and subjecting the dried product to mass spectrometry by a matrix-assisted laser desorption/ionization method to obtain a mass spectrum. The matrix solution is an aqueous solution containing acetonitrile, ethanol, trifluoroacetic acid, and water. A content of the acetonitrile is 30 to 40 vol% based on the matrix solution. A content of the ethanol is 10 to 20 vol% based on the matrix solution. A content of the trifluoroacetic acid is 1 to 3 vol% based on the matrix solution. The matrix solution contains α-cyano-4-hydroxycinnamic acid, 2,5-dihydroxybenzoic acid, or sinapinic acid in a ratio of 5 to 20 mg/ml.

According to the mass spectrometry method of item 5, a matrix solution capable of measuring a mass spectrum with high sensitivity can be provided. Moreover, when the sample is a sample derived from a microorganism, accuracy of determining a microorganism using a mass spectrum can be improved.

(Item 6) In the mass spectrometry method according to item 5, the sample is a sample derived from a microorganism.

When the sample is a sample derived from a microorganism as described in item 6, the accuracy of determining a microorganism using a mass spectrum can be improved.

(Item 7) A storage medium storing database including the mass spectrum obtained by the mass spectrometry method according to item 5 or 6.

According to the storage medium of item 7, database including mass spectra with high sensitivity can be stored, and can be taken out to the outside.

(Item 8) A determination method for determining a type of a microorganism by using database including the mass spectrum obtained by the mass spectrometry method according to item 6.

According to the determination method of item 8, a microorganism can be determined with high accuracy by using the database including a mass spectrum with high sensitivity.

(Item 9) In the determination method according to item 8, the type of a microorganism includes at least one of taxonomic classes of the genotype, the strain, the subspecies, the species, the genus, and the family of a microorganism.

According to the determination method of item 9, accuracy of determining the type of a microorganism as described in item 9 can be improved. In particular, microorganisms with high affinity can be determined.

(Item 10) The determination of a type of a microorganism according to item 8 or 9 includes at least one of classification, identification, and discrimination of the type of a microorganism.

In the determination method according to item 10, the accuracy in classification, identification, and discrimination of the type of a microorganism is probably improved.

It should be regarded that the embodiments disclosed herein are not limiting but illustrative in all points. The scope of the present invention is not limited by the description given above but limited by the scope of appended claims, and is intended to encompass equivalence of the scope of appended claims, and all modifications made within the scope.

### REFERENCE SIGNS LIST

1 Analysis device; 5 Pipette; M Matrix solution; 10 Control unit; 11 Processing part; 12 Storage part; 13 Input/output part; 20 Measurement unit; 21 Ionization part; 22 Ion acceleration part; 23 Mass separation part; 24 Detection part; 111 Device control part; 112 Mass spectrum creation part; 113 Mass spectrum analysis part; 114 Determination part; 131 Input part; 132 Output part; 133 Communication part; 221 Accelerating electrode; 231 Flight tube; P Sample plate; S ion; W well.

## Claims

1. A matrix solution used to be mixed with a sample in a matrix-assisted laser desorption/ionization mass spectrometry method,
wherein the matrix solution is an aqueous solution comprising acetonitrile, ethanol, trifluoroacetic acid, and water,
a content of the acetonitrile is 30 to 40 vol% based on the matrix solution,
a content of the ethanol is 10 to 20 vol% based on the matrix solution,
a content of the trifluoroacetic acid is 1 to 3 vol% based on the matrix solution, and
the matrix solution comprises α-cyano-4-hydroxycinnamic acid, 2,5-dihydroxybenzoic acid, or sinapinic acid in a ratio of 5 to 20 mg/ml.

2. The matrix solution according to claim 1,
wherein the content of the acetonitrile is 33 to 37 vol% based on the matrix solution, and
the content of the ethanol is 13 to 17 vol% based on the matrix solution.

3. The matrix solution according to claim 1 or 2, wherein the α-cyano-4-hydroxycinnamic acid is comprised in a ratio of 8 to 10 mg/ml.

4. The matrix solution according to claim 1 or 2, wherein the sample is a sample derived from a microorganism.

5. A mass spectrometry method comprising:
mixing a sample and a matrix solution;
drying the matrix solution having been mixed with the sample on a sample plate to form a dried product; and
subjecting the dried product to mass spectrometry by a matrix-assisted laser desorption/ionization method to obtain a mass spectrum,
wherein the matrix solution is an aqueous solution containing acetonitrile, ethanol, trifluoroacetic acid, and water,
a content of the acetonitrile is 30 to 40 vol% based on the matrix solution,
a content of the ethanol is 10 to 20 vol% based on the matrix solution,
a content of the trifluoroacetic acid is 1 to 3 vol% based on the matrix solution, and
the matrix solution comprises α-cyano-4-hydroxycinnamic acid, 2,5-dihydroxybenzoic acid, or sinapinic acid in a ratio of 5 to 20 mg/ml.

6. The mass spectrometry method according to claim 5, wherein the sample is a sample derived from a microorganism.

7. A storage medium storing database including the mass spectrum obtained by the mass spectrometry method according to claim 5 or 6.

8. A determination method for determining a type of a microorganism by using database including the mass spectrum obtained by the mass spectrometry method according to claim 6.

9. The determination method according to claim 8, wherein the type of a microorganism includes at least one of taxonomic classes of a genotype, a strain, a subspecies, a species, a genus, and a family of a microorganism.

10. The determination method according to claim 8 or 9, wherein the determination of the type of a microorganism includes at least one of classification, identification, and discrimination of the type of a microorganism.
